# EUROPÄISCHE PATENTANMELDUNG

(11) **EP 0 732 077 A1**
(43) Veröffentlichungstag der Anmeldung: **18.09.1996**
(21) Anmeldenummer: 94119811.1
(22) Anmeldetag: 20.12.1994
(51) Int. Cl.: A61B 5/14, A61M 25/06

(54) **Vorrichtung zum Punktieren von Blutgefässen mit einer Kanüle**

(71) Anmelder: v. Froreich, André, Dr., D-21149 Hamburg (DE)
(72) Erfinder: v. Froreich, André, Dr., D-21149 Hamburg (DE)
(74) Vertreter: von Raffay, Vincenz, Dipl.-Ing.

(57) **Zusammenfassung**

Die Vorrichtung zum Punktieren von Blutgefäßen mit einer Kanüle mit zwei zugespitzten Rohrenden, ist so ausgebildet, daß die beiden Rohrenden (2 und 3) in einem Winkel von ungefähr 90° zueinander in einem Kunststoffblock (11) befestigt sind, der eine Standfläche (12) zur Auflage auf der zu punktierenden Person aufweist. Das eine Rohrende (2) verläuft parallel zu der Standfläche (12), wohingegen das andere um ca. 90° nach oben abgewinkelt ist. Es sind zwei Ausführungsformen möglich. Bei der einen Ausführungsform sind die Rohrenden (2 und 3) Teil eines einstückigen Kanülenrohres (1). Bei der anderen Ausführungsform sind die Rohrenden getrennt und durch einen Hohlraum in dem Kunststoffblock verbunden. Es ist eine Ausführung zur häufigen Wiederverwendung und zum einmaligen Einsatz möglich. Durch die Abwinkelung und Anbringung in dem Kunststoffblock in Verbindung mit der großen Standfläche wird die medizinische und die technische Handhabung vereinfacht.

## Beschreibung

Die Erfindung betrifft eine Vorrichtung nach dem Oberbegriff des Patentanspruches 1.

In der Medizin ist die Schaffung eines Zugangs zum Blutkreislauf von eminenter Bedeutung. Das am häufigsten verwendete Verfahren ist die Punktion eines Gefäßes durch Einführen eines dünnen Metallrohres oder Kanüle in das Lumen des Gefäßes, durch das Blutproben entnommen, Medikamente injiziert oder Bluttransfusionen vorgenommen werden können. Die Kanüle läuft an ihrem vorderen Ende spitz zu und ist angeschliffen, um mit möglichst geringem Kraftaufwand und Schmerzen für den Patienten durch Haut, Unterhautgewebe und Gefäßwand in das Blutgefäßlumen dringen zu können.

Da unterschiedliche Kanülenstärken verwendet werden und meist mehrere Instrumente (unterschiedliche Spritzen, Schläuche) im Rahmen einer Punktion abwechselnd mit derselben Kanüle verbunden werden müssen, ist eine lösbare Verbindung zwischen Kanüle und anzuschließenden Instrumenten unentbehrlich. Zu diesem Zweck besitzt eine Kanüle im konventionellen Fall an ihrem rückwärtigen Ende einen festverbundenen Innenkonus aus Metall oder Kunststoff, an den Spritzen zur Blutentnahme bzw. zur Verabreichung von Medikamenten oder auch Schläuche zur Verabreichung von Infusionen und Bluttransfusionen angeschlossen werden können. Eine konventionelle Kanüle wird vor der Gefäßpunktion auf den Konus einer Spritze geklemmt. Bei der konventionellen Gefäßpunktion dient die Spritze als Halter und Führungsinstrument für die Kanüle.

Eine Variante der konventionellen Kanüle ist die sogenannte Flügelkanüle. Diese besitzt in Höhe des Innenkonus am rückwärtigen Ende eine oder zwei seitlich abstehende Flächen als Handgriffe zur Führung der Kanüle. Die seitlich abstehenden Flächen können senkrecht zur Kanülenachse am Konus befestigt sein und haben in der Regel eine ohrähnliche Form. Eine Flügelkanüle kann mit Hilfe der seitlichen Griffflächen ohne andere Hilfsmittel zur Punktion verwendet werden. Flügelkanülen werden wegen ihrer seitlichen Stabilisierung auf der Unterlage durch die Griffflächen vorwiegend für den längerfristigen Gefäßzugang benutzt (sogenannte Dauerkanülen) wie z.B. bei Infusionen und Bluttransfusionen.

Wegen der zunehmenden Furcht vor Infektion mit Hepatitis- und HIV-Viren wurden neue Punktionssysteme entwickelt. Diese neuen Punktionssysteme zeichnen sich durch die Verwendung von Ventilmechanismen und durchstechbare Membranen aus, die das Austreten von Blut während des Wechsels von anzuschließenden Instrumenten verhindern. Die Kanülenform wurde entsprechend angepaßt. Eine Sonderform der Kanüle, die für die Blutentnahme mit Ventil- und Membransystemen weite Verbreitung gefunden hat, ist an beiden Enden spitz zulaufend und scharf angeschliffen. Sie kann dadurch an ihrem rückwärtigen Ende elastische Abschlußmembranen oder -Stopfen von Gefäßen für die Aspiration von Blut durchbohren.

Ein Abnahmesystem mit einer solchen Kanüle ist in der deutschen Offenlegungsschrift 1 812 742 beschrieben. Diese Kanüle wird mit Hilfe eines Halters, in dem sie durch ein Gewinde befestigt ist, in das Blutgefäßlumen geschoben. Um einen spontanen Blutaustritt am rückwärtigen Ende zu vermeiden, ist das rückwärtige Ende mit einer über das rückwärtige Ende der Kanüle übergezogenen Gummikappe verschlossen. Nach erfolgreicher Gefäßpunktion wird auf das hintere Ende der Kanüle ein evakuiertes Gefäß aufgeschoben, das an seinem vorderen Ende mit einem elastischen Stopfen zur Kanüle hin abgeschlossen ist. Durch Druck des Gefäßes auf die rückwärtige Kanülenspitze werden zuerst die Gummikappe der Kanüle und anschließend der elastische Stopfen des evakuierten Gefäßes von der Kanüle durchbohrt und durch Verbindung des evakuierten Gefäßinnenraums mit dem Kanülenlumen Blut aus dem punktierten Blutgefäß angesaugt. Nach erfolgter Füllung kann das evakuierte Gefäß abgezogen werden, wobei sich die Gummikappe aufgrund ihrer Elastizität wieder über die Kanülenspitze schiebt und diese gegen ungewollten Blutaustritt abdichtet. Danach kann der Vorgang bei liegender Kanüle mit anderen evakuierten Gefäßen beliebig wiederholt werden.

Dieses Blutabnahmesystem hat inzwischen weltweit große Verbreitung gefunden und wird von zahlreichen Herstellern vertrieben. Obwohl Vorschläge zur Verbesserung dieses Abnahmesystems bekannt sind, hat sich an der heute allgemein verwendeten Form im Vergleich zum o.g. Beispiel nichts Grundlegendes geändert. Einen Schwerpunkt innerhalb dieser Bemühungen bildet das Bestreben, den Eintritt der Kanülenspitze in das Gefäßlumen sichtbar zu machen, indem eine Art kleines Schauglas in den Verlauf der Kanüle eingeschaltet wird. In die Kanüle eintretendes Blut wird beim Passieren des Schauglases sichtbar und gibt der abnehmenden Person die Gewißheit, ein Blutgefäß punktiert zu haben. Alle diesbezüglichen Vorschläge haben sich jedoch bisher in der Praxis nicht durchsetzen können.

Die konventionelle Kanüle mit einem fest verbundenen Innenkonus an ihrem rückwärtigen Ende als lösbare Verbindungsmöglichkeit für unterschiedliche Spritzen oder Schläuche ist einfach, kostengünstig und mechanisch zuverlässig. Eine Kanüle, die mittels Innenkonus auf eine Spritze geklemmt ist, kann durch eine solche Verlängerung des Hebelarms eine kraft- und gefühlvolle Handhabung erlauben. Da der Konus der Spritze bei Spritzen größeren Durchmessers exzentrisch angebracht werden kann, kann der Anstich des Blutgefäßes flach erfolgen, d.h. annähernd parallel zur Gefäßachse. Die scharfe Spitze der Kanüle läuft dadurch weniger Gefahr, die gegenüberliegende Gefäßwand zu durchbohren und ein Hämatom (Blutaustritt in das umliegende Gewebe) zu erzeugen, was besonders bei der Punktion kleinerer Gefäße wichtig ist. Ein weiterer Vorteil dieser Technik ist die Möglichkeit, den Eintritt der Kanülenspitze in das Blutgefäß an einem Bluteintritt in den Spritzenkonus feststellen zu können, der in aller Regel durchscheinend ist.

Der Nachteil von Kanülen mit einfachem Konus ist der Austritt von Blut bei einem Wechsel der Spritzen und damit die Gefahr einer Kontamination der abnehmenden Person mit infiziertem Blut. Dieser Nachteil wird als so gravierend angesehen, daß diese Technik allmählich zugunsten kontaminationsfreier Punktionssysteme verlassen wird.
Weil im Zusammenhang mit konventionellen Kanülen nur Spritzen zur Blutabnahme verwendet werden können, ergibt sich bei konventioneller Technik der Nachteil, daß bei der Aspiration von Blut eine relativ hohe axiale Kraft auf den Spritzenkolben ausgeübt wird, der durch Festhalten des Spritzenzylinders ausgeglichen werden muß. Schwankungen in der Summe dieser Kräfte führen zu axialen Verschiebungen der Kanülenspritze im Gefäßlumen, verbunden mit der Gefahr des Herausgleitens aus dem Blutgefäß, des Durchbohrens oder des Verletzens der Blutgefäßwandung mit der angeschliffenen Spitze der Kanüle.
Beim Wechsel der Instrumente am Innenkonus der Kanüle ist die Ausübung einer Kraft auf die im Gefäßlumen liegende Kanüle ein weiterer Nachteil, der, wie bei der Aspiration von Blut mit dem Spritzenkolben, zu unerwünschten Bewegungen der liegenden Kanüle führen kann. Um beispielsweise einen festen Sitz im Konus zu erreichen, und um diesen wieder zu lösen, muß sowohl eine Dreh- wie eine axiale Schubbewegung in dem Innenkonus ausgeführt werden, der meist mit Kraftaufwand verbunden ist. Dies führt wiederum zu ungewollten Dreh- und Schubbewegungen an der Kanülenspitze mit den oben bereits genannten Auswirkungen. Deshalb ist ein Instrumentenwechsel eine häufige Ursache für Hämatombildungen im Anschluß an eine korrekte Gefäßpunktion.

Die vorgenannten Nachteile gelten auch in Bezug auf die Flügelkanüle, mit der Einschränkung, daß beim Instrumentenwechsel durch die seitlich abstehenden Griffe eine Stabilisierung gegen ein Verdrehen der liegenden Kanüle erreicht wird.
Ein Vorteil der Flügelkanüle liegt in der Anordnung der seitlichen Griffflächen, die im Gegensatz zu einer Spritze als Führungsinstrument eine ergonomisch bessere Anordnung darstellen. Eine seitliche Grifffläche, die senkrecht zur Kanülenachse angeordnet ist, kann mit einer natürlichen Handhaltung zwischen Daumen und Zeigefinger gefaßt werden, während eine Spritze, die in axialer Richtung zur Kanüle angeordnet ist, ein Verdrehen der Hand um 90° nach außen voraussetzt, um zwischen Daumen, Zeigefinger und Ringfinger gefaßt zu werden. Es ist einleuchtend, daß eine natürliche Handhaltung während einer Gefäßpunktion eine sehr viel bessere Kontrolle der Kraft aus dem Handgelenk heraus erlaubt und sehr viel mehr Feingefühl vermittelt als eine um 90° gedrehte Handhaltung.

Das in der deutschen Offenlegungsschrift 1 812 742 beschriebene Blutabnahmesystem hat gegenüber dem konventionellen System den Vorteil des Kontaminationsschutzes für die das Blut abnehmende Person. Durch die Verwendung von evakuierten Gefäßen, die selbsttätig Blut ansaugen, entfällt die Aspiration durch eine Kolbenbewegung und bietet dadurch entsprechende Zeitvorteile. Erkauft werden diese Vorteile allerdings mit einigen Nachteilen gegenüber der konventionellen Methodik.
Der Halter, in dem die Kanüle durch eine Schraubverbindung befestigt wird, ist kürzer als eine Spritze und ebenso axial zur Kanüle angeordnet. Die Führung der Kanüle ist mit Hilfe des Halters wegen seiner kleineren Größe ergonomisch nicht so günstig wie z.B. mit Hilfe einer Spritze. Die Kanüle wird bei allen marktgängigen Produkten zentral in den röhrenförmigen Halter eingeschraubt. Dies vergrößert die Steilheit des Einstichs mit der Kanüle und vergrößert damit die Gefahr des Durchstechens des zu punktierenden Gefäßes. Obwohl seit langem bekannt, hat sich eine exzentrische Positionierung der Kanüle im Halter zur Verringerung des Einstechwinkels, möglicherweise aus Kosten- und fertigungstechnischen Gründen, nicht durchgesetzt. Die Stabilität der Kanüle ist aus dem vorgenannten Grund bei liegender Kanüle geringer als z.B. bei der konventionellen Technik und bedeutend geringer als bei der Flügelkanüle. Diese Instabilität erhöht die nachstehenden Gefahren beim Wechsel der evakuierten Gefäße.
Beim Einschieben des evakuierten Gefäßes in den Halter tritt bei der Perforation des elastischen Verschlusses am vorderen Ende des evakuierten Gefäßes durch das rückwärtige Ende der Kanüle ein mechanischer Widerstand auf, der eine Kraftausübung in axialer Richtung erfordert. Diese Kraft wird durch Festhalten des Kanülenhalters/Führungshülse aufgefangen und führt bei schwankender Differenz aus Kraft und Gegenkraft zu axialen Bewegungen der Kanülenspitze im Gefäßlumen mit der Gefahr des Herausgleitens aus dem Gefäß, Verletzen oder Durchbohren desselben und der Erzeugung von Hämatomen.
Ein größerer Nachteil dieses Verfahrens ist das Fehlen einer Möglichkeit zur Feststellung des Bluteintritts in die Kanüle bei der Punktion. Die Bedeutung dieses Problems wird aus einer Vielzahl von Lösungsvorschlägen ersichtlich, die jedoch bisher in handelsüblichen Systemen, möglicherweise aus Kosten- und Fertigungsgründen, nicht verwirklicht wurden.

Die vorgenannten Nachteile sind der Grund für die Zurückhaltung zahlreicher Anwender gegenüber dieser ansonsten kostengünstigen, schnellen und hygienischen Abnahmetechnik.

Die Erfindung hat die Aufgabe,
1. eine ergonomisch optimale Führung der Punktionskanüle zu gewährleisten;
2. ein Einstechen der Punktionskanüle unter einem flachen Winkel zu ermöglichen,
3. den Bluteintritt in die Kanüle sichtbar zu machen,
4. eine einwandfreie Fixierung der im Gefäßlumen liegenden Kanüle auf ihrer Unterlage zu ermöglichen,
5. die erforderlichen Kräfte für den Instrumenten- oder Gefäßwechsel ohne Beeinflussung der Kanülenlage abzuleiten,
6. diese Aufgaben ohne nennenswerte Verteuerung des Punktionsverfahrens, d.h. kostengünstig, zu erfüllen,
7. die Verwendung handelsüblicher Ventilmechanismen, evakuierter Gefäße, Anschlußgewinde und Schutzhülsen für die Kanüle zu ermöglichen.

Wesentliche Punkte der vorstehend definierten Aufgabe werden durch das Kennzeichen des Anspruches 1 gelöst.

Weitere vorteilhafte Lösungen und Einzellösungen der definierten Aufgabe sind Gegenstand der Unteransprüche.

Die erfindungsgemäße Vorrichtung ist im wesentlichen durch zwei Merkmale, nämlich die abgewinkelten, zugespitzten Rohr- oder Kanülenenden und die Befestigung dieser Rohrenden in einem hierfür ausgebildeten Kunststoffblock, gekennzeichnet. Das zum Punktieren vorgesehene Rohrende verläuft im wesentlichen parallel zur Standfläche, so daß, wie eingangs beschrieben, ein ungefährdetes Punktieren des Patienten möglich ist. Das abgewinkelte Rohrende ist gut zugänglich, um entweder die gewünschte Blutentnahme in der unterschiedlichsten Weise durchzuführen oder aber dem Patienten Flüssigkeit zuzuführen. Der Kunststoffblock ermöglicht bzw. erleichtert die verschiedenen Handhabungen.

Grundsätzlich ist es möglich, das Kanülenrohr durchgehend, d.h. einstückig auszubilden oder aber in zwei Teilen, wobei im letzteren Fall in dem Kunststoffblock ein Hohlraum vorgesehen ist, in den die beiden Rohrenden führen. Die Befestigung der Rohrenden in dem Kunststoffblock kann entweder durch bauliche Maßnahmen, d.h. mechanisch oder durch Umspritzen, d.h. dauerhaft, erfolgen. Die zuletzt genannte Befestigung eignet sich besonders für einmalig verwendbare Vorrichtungen.

Die Anbringung von Griffflächen an dem Kunststoffblock, die in unterschiedlichen Ebenen möglich ist, erleichtert die verschiedenen Handhabungen, insbesondere das Punktieren und den Einsatz des anderen, abgewinkelten Rohrendes bei einer Blutentnahme oder bei einer Zuführung von Flüssigkeit.

Im folgenden wird die Erfindung unter Hinweis auf die Zeichnung anhand verschiedener Ausführungsbeispiele näher erläutert.

Es zeigt:
- Fig. 1: eine auseinandergezogene, perspektivische Ansicht einer Ausführungsform einer Vorrichtung nach der Erfindung ohne Kunststoffblock;
- Fig. 2: eine perspektivische Ansicht des Kunststoffblockes für die in Fig. 1 dargestellte Ausführungsform;
- Fig. 3: eine perspektivische Ansicht der in den Fig. 1 und 2 dargestellten wesentlichen Teile von unten;
- Fig. 4: einen Teilschnitt durch eine Ausführungsform mit einem Adapter zur Befestigung eines Vakuumaufnahmegefäßes;
- Fig. 5: eine auseinandergezogene, perspektivische Darstellung der Ausführungsform nach Fig. 4; und
- Fig. 6: einen Schnitt durch eine abgewandelte Ausführungsform, bei der die Rohrenden durch den den Kunststoffblock bildenden Kunststoff umspritzt sind, wobei wiederum ein Adapter für ein Vakuumaufnahmegefäß vorgesehen ist.

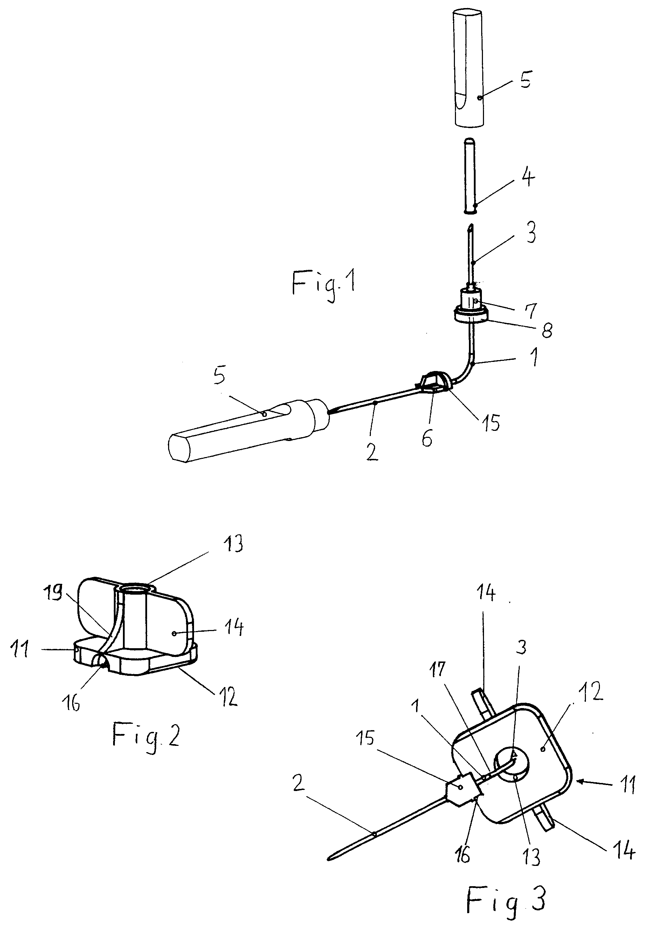

Die in Fig. 1 dargestellte Vorrichtung besteht aus einem um ca. 90° abgewinkelten Kanülenrohr 1, dessen Rohrenden 2 und 3 zugespitzt sind. Das Rohrende 2 dient der Punktion des zu behandelnden Patienten, wohingegen das Rohrende 3 für die Blutentnahme oder für die Zuführung von Flüssigkeit vorgesehen ist. Bei dieser Ausführungsform sind die Rohrenden 2 und 3 Teil eines einzigen, einstückigen Kanülenrohres 1.

Auf dem Rohrende 2 befindet sich ein Anschlußstück 15 mit Aufsteckführungen für eine Schutzhülle 5. Auf das Rohrende 3 ist eine Führungshülse 7 aufgeschoben und fixiert, die einen Flansch 8 größeren Durchmessers aufweist.

In Fig. 2 ist der Kunststoffblock 11 dargestellt. Dieser weist eine Aussparung 16 zur Aufnahme eines Abschnittes des Anschlußstückes 15 auf. Die unten liegende Standfläche des Kunststoffblockes 11 ist mit 12 bezeichnet. Senkrecht zu der Standfläche 12 ist eine Durchgangsbohrung 13 vorgesehen. Griffflächen 14 können so orientiert sein, wie in Fig. 2 dargestellt. Zusätzlich zu den Griffflächen können noch Verstärkungsrippen 19 vorgesehen sein. Die Griffflächen können aber auch in einer anderen Ebene, beispielsweise in derjenigen der Verstärkungsrippen 19 oder senkrecht zu diesen, verlaufen.

Beim Betrachten der Fig. 3 wird deutlich, wie das Anschlußstück 15 mit dem Rohrende 2 in der Aussparung 16 im Bereich der Standfläche 12 des Kunststoffblockes 11 befestigt ist. Die Aufsteckführungen 6 des Anschlußstückes 15 bleiben zur Aufnahme der Schutzhülle 5 frei. Für das Kanülenrohr 1, das, ausgehend von dem Rohrende 2, durch das Anschlußstück 15 hindurchgeht, ist in der Standfläche 12 ein Schlitz 17 vorgesehen. Das Kanülenrohr 1 ist um ca. 90° abgebogen und verläuft durch die Durchgangsöffnung 13 nach oben.

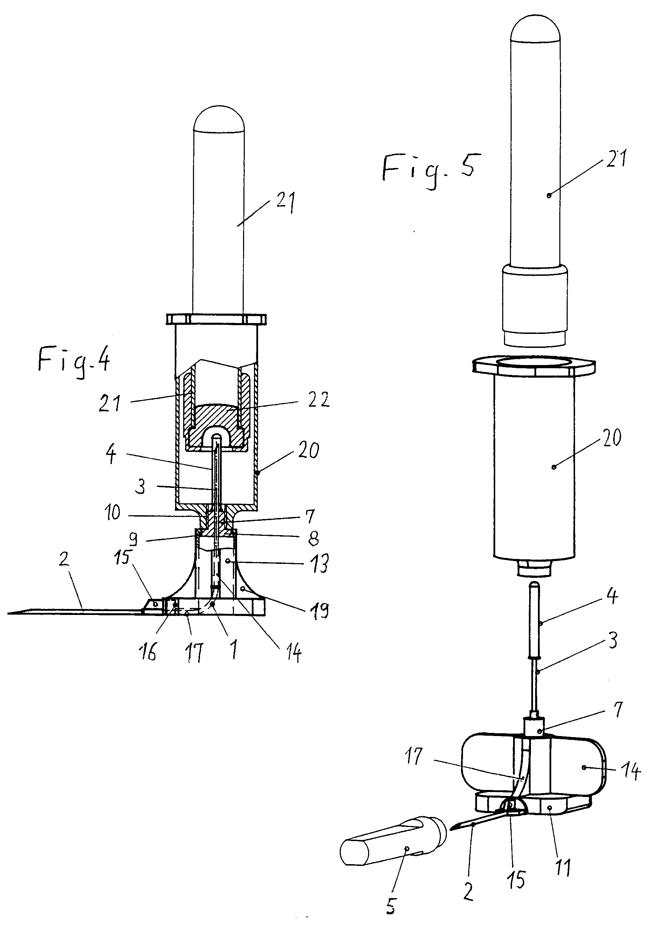

In Fig. 4 ist dargestellt, wie das Rohrende 3 mit Hilfe der Führungshülse 7 in dem Kunststoffblock 11 befestigt ist. Die Führungshülse 7 mit ihrem Flansch 8, der bei 9 noch eine Flanschstufe etwas größeren Durchmessers aufweist, ist auf das Rohrende 3 aufgeschoben und wird dann von unten in den Kunststoffblock in die Durchgangsöffnung 13 eingeführt, bis die Flanschstufe 9 größeren Durchmessers an einem entsprechend dimensionierten Innenflansch am oberen Ende der Durchgangsöffnung 13 des Kunststoffblockes zur Anlage gelangt. Wenn dann ein Adapter 20 oder ein entsprechendes Teil mit einem Innengewinde 10 auf das Außengewinde 10 der Führungshülse 7 aufgeschraubt wird, werden die beiden Flansche gegeneinander gezogen, so daß eine feste Verbindung entsteht.

Der Adapter 20 dient gleichzeitig der Anbringung eines Vakuumaufnahmegefäßes 21, das an seiner unteren Stirnfläche mit einer Dichtung 22 versehen ist. Auf dem Rohrende 3 befindet sich eine Gummikappe 4 aus durchsichtigem oder halbdurchsichtigem Material. Wenn das Vakuumaufnahmegefäß zur Blutaufnahme eingesetzt werden soll, wird dieses in dem Adapter 20 in Richtung auf den Kunststoffblock 11 nach unten geschoben, so daß die Gummikappe 4 ziehharmonikaartig zusammengeschoben und gleichzeitig diese und die Dichtung 22 durchstochen wird. Nach der Blutentnahme kann das Vakuumgefäß nach oben abgezogen werden. Die elastischen Materialeigenschaften der Dichtung 22 sorgen für einen dichten Verschluß. Die Gummikappe 4 nimmt im wesentlichen ihre Ausgangsstellung wieder ein und schließt das offene Rohrende 3 wieder ab.

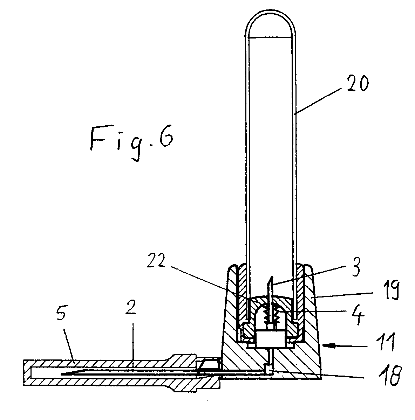

In Fig. 6 ist eine andere Ausführungsform dargestellt, bei der es sich um eine einmal zu verwendende Vorrichtung, d.h. um eine sog. Einweg-Vorrichtung handelt. Bei dieser Ausführungsform sind zwei getrennte Rohrenden 2 und 3 vorgesehen, die von dem Material des Kunststoffblockes 11 umgeben sind und dauerhaft mit dem Kunststoffblock verbunden sind. Ein Hohlraum 18 in dem Kunststoffblock verbindet die beiden Rohrenden. In diesem Bereich kann der Kunststoffblock durchsichtig sein, so daß erkennbar ist, ob sich Blut oder eine andere Flüssigkeit in der Kanüle befindet.

Es ist erkennbar, daß der Kunststoffblock 11 etwas anders gestaltet ist, als derjenige der Ausführungsform nach den Fig. 1 bis 5. Zwischen den Griffflächen 14 bzw. den Verstärkungsrippen 19 ist eine Vertiefung vorgesehen, die - wie in Fig. 6 dargestellt - das Vakuumaufnahmegefäß 20 aufnimmt. Es ist aber auch möglich, anstelle des Vakuumaufnahmegefässes lediglich eine Schutzhülle 5 aufzuschieben, d.h. zwischen den Griffflächen 14 und den Verstärkungsrippen 19 anzuordnen.

Grundsätzlich geben die seitlichen Griffflächen 14 und das nach vorn aus der Standfläche 12 des Kunststoffblockes 11 austretende Rohrende 2 bei sämtlichen Ausführungsformen der Kanülen-Block-Einheit einen festen Halt auf der Unterlage, da die Griffflächen durch ihre Anordnung und Gestaltung eine Vergrößerung der Standfläche bewirken und durch Eindrücken in die Unterlage eine Verschiebung in axialer Richtung verhindern. Sie sind gleichzeitig eine Absicherung gegen Verdrehen des Kanülenrohres um seine Achse. Durch diese Eigenschaften wird die Bewegung des Kanülenrohres im Gefäßlumen sowohl in axialer Richtung als auch eine Drehung um die eigene Achse verhindert.

Durch senkrechte Führung des abgewinkelten Rohrendes 3 können Kräfte, die im Zusammenhang mit einem Wechsel von Vakuumaufnahmegefäßen 20 auftreten, nicht mehr die im Gefäßlumen des punktierten Blutgefäßes liegende Rohrspitze in axialer Richtung beeinflußen. Sie werden vielmehr senkrecht auf die Unterlage geleitet und führen dadurch im Gegenteil zur Stabilisierung des liegenden Kanülenrohres.

Die Vorrichtung nach der Erfindung ist kostengünstig herstellbar, weil die gesamten Zubehör- und Anschlußteile , wie Gummikappe, Vakuumentnahmegefäß, Anschlußgewinde, Adapter und Schutzhüllen, unverändert aus dem Handel übernommen werden können. Der Mehraufwand beschränkt sich praktisch auf das Biegen des Kanülenrohres um 90° bzw. die zweiteilige Ausbildung des Kanülenrohres und die Herstellung eines einfachen, kostengünstigen Kunststoffblockes.

### Bezugszeichenliste

- 1: Kanülenrohr
- 2: Rohrende zum Punktieren
- 3: Rohrende, abgewinkelt
- 4: Gummikappe
- 5: Schutzhülle
- 6: Aufsteckführungen
- 7: Führungshülse
- 8: Flansch
- 9: Flanschstufe
- 10: Innen- und Außengewinde
- 11: Kunststoffblock
- 12: Standfläche
- 13: Durchgangsbohrung
- 14: Grifffläche
- 15: Anstoßstücke
- 16: Aussparung
- 17: Aufnahmeschlitz
- 18: Hohlraum
- 19: Verstärkungsrippe
- 20: Adapter
- 21: Vakuumaufnahmegefäß
- 22: Dichtung

## Patentansprüche

1. Vorrichtung zum Punktieren von Blutgefäßen mit einer Kanüle, deren Rohrenden zugespitzt sind, dadurch gekennzeichnet, daß die beiden Rohrenden (2,3) in einem Winkel von ungefähr 90° zueinander und in einem Kunststoffblock befestigt sind, der eine Standfläche (12) zur Auflage auf der zu punktierenden Person aufweist, wobei das für die Punktion vorgesehene Rohrende (2) parallel zu der Standfläche und das andere, um ca. 90° abgewinkelte Rohrende (3), ungefähr senkrecht hierzu verläuft.

2. Vorrichtung nach Anspruch 1, dadurch gekennzeichnet, daß ein die beiden zugespitzten Rohrenden (2,3) aufweisendes, durchgehendes und um ca. 90° gebogenes Kanülenrohr (1) in dem Kunststoffblock (11) befestigt ist.

3. Vorrichtung nach Anspruch 1, dadurch gekennzeichnet, daß jedes zugespitzte Rohrende (2,3) an einem Kanülenrohr ausgebildet ist, die in einem Winkel von ca. 90° zueinander in dem Kunststoffblock (11) befestigt und durch einen Hohlraum (18) in dem Kunststoffblock miteinander verbunden sind.

4. Vorrichtung nach einem oder mehreren der vorstehenden Ansprüche, dadurch gekennzeichnet, daß an dem Kunststoffblock (11) in der Ebene des um ca. 90° abgewinkelten Rohrendes (3) und senkrecht zu der Standfläche (12) entweder senkrecht zu dem für die Punktion vorgesehenen Rohrende (2) Griffflächen (14) vorgesehen sind.

5. Vorrichtung nach einem oder mehreren der vorstehenden Ansprüche, dadurch gekennzeichnet, daß der Kunststoffblock (11) an der Unterseite der Standfläche eine Aufnahme (17) für das zur Punktion vorgesehene Rohrende (2) und eine Durchgangsöffnung (13) für das andere, um ca. 90° abgewinkelte Rohrende (3) aufweist.

6. Vorrichtung nach Anspruch 5, dadurch gekennzeichnet, daß auf das um 90° abgewinkelte Rohrende (3) eine Führungshülse (7) aufgeschoben ist, die in die Durchgangsöffnung (13) für dieses Rohrende einsetzbar ist.

7. Vorrichtung nach Anspruch 5 oder 6, dadurch gekennzeichnet, daß das für die Punktion vorgesehene Rohrende (2) ein Anschlußstück (15) aufweist, das in eine Aussparung (16) in dem Kunststoffblock (11) einsetzbar ist, und auf das ggf. eine Schutzhülle (5) aufsetzbar ist.

8. Vorrichtung nach Anspruch 6, dadurch gekennzeichnet, daß auf den Kunststoffblock (11), vorzugsweise auf die Führungshülse (7) ein Adapter (20) für ein Vakuumaufnahmegefäß (21) oder dergleichen aufschraubbar ist.

9. Vorrichtung nach einem oder mehreren der Ansprüche 1 bis 4, dadurch gekennzeichnet, daß die Rohrenden (2,3) durch Umspritzen, Einkleben, Einpressen oder dergleichen dauerhaft mit dem Kunststoffblock verbunden sind.

10. Vorrichtung nach Anspruch 3, dadurch gekennzeichnet, daß das Material des Kunststoffblockes (11) im Bereich des Hohlraumes (18) durchsichtig ist.
